# EUROPEAN PATENT APPLICATION

(11) **EP 4 732 756 A1**
(43) Date of publication of application: **29.04.2026**
(21) Application number: 24208561.1
(22) Date of filing: 24.10.2024
(51) Int. Cl.: A61B 5/00, H03M 7/30

(54) **DATA SIGNAL REPRESENTING A BIOMEDICAL WAVEFORM SIGNAL**

(71) Applicant: Koninklijke Philips N.V., 5656 AG Eindhoven (NL)
(72) Inventor: PERRONE, Antonio Luigi, Eindhoven (NL); JELFS, Sam Martin, Eindhoven (NL); OOMEN, Arnoldus Werner Johannes, Eindhoven (NL); DE BONT, Fransiscus Marinus Jozephus, Eindhoven (NL)
(74) Representative: Philips Intellectual Property & Standards

(57) **Abstract**

An apparatus comprises a receiver (201) receiving a biomedical waveform time domain signal and a circuit (203) arranging samples of the biomedical signal into an N-dimensional data structure (N≥2). A compression circuit (205) generates compressed data by applying a compression process to the N-dimensional data structure where the compression process includes an N-dimensional transform coding. A data signal generator (207) generates the data signal to include the compressed data, and an adapter (209) adapts the arranging of samples of the biomedical signal into the structure dependent on a property, such as for example a periodicity, of the biomedical waveform time domain signal. A complementary apparatus is arranged to perform the complementary operations to reconstruct the biomedical waveform time domain signal.

## Description

### FIELD OF THE INVENTION

The invention relates to compression and/or decompression of biomedical waveform signal(s), and in particular, but not exclusively, of an electrocardiography (ECG), electroencephalography (EEG), electromyography (EMG), or photoplethysmography (PPG) signal.

### BACKGROUND OF THE INVENTION

The capturing, storing, distributing, and processing of biomedical data has become increasingly important in recent decades as recording such signals has progressed from being performed almost exclusively for short term measurements in dedicated clinical environments, such as hospitals, to becoming increasingly prevalent in the personal space with biomedical measurements often being made continuously over very long periods. For example, the cardiology field has evolved from mainly considering short heart rate waveforms collected in a clinician's office to frequently using chest-worn outpatient devices that are routinely worn for multiple days, and which collect continuous data for long durations.

Consequently, the requirements for data storage, distribution, and processing have grown exponentially. Accordingly, there is a desire to effectively represent captured biomedical waveform signals to facilitate and reduce the requirements for storing, distributing, and/or processing the signals. However, whereas various proprietary approaches have been proposed for encoding or compressing biomedical waveform signals used by various manufacturers, current approaches tend to be suboptimal and tend to not provide ideal performance. Further, current approaches tend to be complex and/or be targeted on specific biomedical waveform signals resulting in specific requirements and limited application.

Hence, an improved approach would be advantageous. In particular, an approach allowing increased flexibility, improved performance, increased quality, reduced complexity, reduced data rate, increased accuracy/reduced distortion of encoding/decoding/compressing/decompressing operations, reduced storage requirements, reduced bandwidth/data rate for biomedical waveform signals, reduced computational load, facilitated implementation, and/or improved performance would be advantageous.

### SUMMARY OF THE INVENTION

Accordingly, the invention seeks to preferably mitigate, alleviate or eliminate one or more of the above-mentioned disadvantages singly or in any combination.

According to an aspect of the invention, there is provided an apparatus for generating a data signal representing a biomedical waveform time domain signal, the apparatus comprising: a receiver arranged to receive the biomedical waveform time domain signal; a circuit arranged to arrange/map samples of the biomedical waveform time domain signal into an N-dimensional data structure, N being no less than two; a compression circuit arranged to generate compressed data representing the biomedical waveform time domain signal by applying a compression process to the N-dimensional data structure, the compression process including an N-dimensional transform coding; a data signal generator arranged to generate the data signal to include the compressed data; and an adapter arranged to adapt the arranging/mapping of samples of the biomedical waveform time domain signal into the N-dimensional data structure dependent on a property of the biomedical waveform time domain signal.

The invention may allow advantageous decompression of a biomedical waveform signal and may support a more efficient and/or accurate compression/decompression operation. The invention may allow a reduced bandwidth/data rate for compressed data representing a biomedical waveform signal. In many embodiments, the storage requirements and/or required communication bandwidth may be reduced substantially. The approach may allow efficient and practical implementation and/or may reduce complexity and processing/computational requirements.

The biomedical waveform signal may specifically be an electrocardiography (ECG), electroencephalography (EEG), electromyography (EMG), or photoplethysmography (PPG) signal. The biomedical waveform signal may also be an audible signal originating from a measurement on a test subject, such as a heartbeat signal, a breathing signal, or a voice signal, acquired by e.g. an electronic stethoscope or another electronic capturing device suitable for capturing such signals.

In some embodiments, the adapter may be arranged to adapt the compression process and/or the transform coding in dependence on a property of the biomedical waveform time domain signal.

The N-dimensional transform coding may be applied to the N-dimensional data structure (potentially following processing/modification of the N-dimensional data structure).

According to an optional feature of the invention, the property of the biomedical waveform time domain signal is a periodicity property of the biomedical waveform time domain signal.

According to an optional feature of the invention, the adapter is arranged to adapt a property of the N-dimensional data structure dependent on the property of the biomedical waveform time domain signal.

In some embodiments, the property of the N-dimensional data structure is a size of the N-dimensional data structure for at least one dimension.

In some embodiments, the property of the N-dimensional data structure is a number of dimensions of the N-dimensional data. In some embodiments, the property of the N-dimensional data structure is N.

According to an optional feature of the invention, the adapter is arranged to adapt a mapping of the samples of the biomedical waveform time domain signal into the N-dimensional data structure dependent on the property of the biomedical waveform time domain signal.

According to an optional feature of the invention, the circuit is arranged to map data samples of a segment of the biomedical waveform time domain signal into a slice of elements of a first dimension of the N-dimensional data structure.

A segment may correspond to the samples for a time interval of the biomedical waveform time domain signal.

In some embodiments, the data circuit is arranged to map data samples of a second segment of the biomedical waveform time domain signal into a further slice of the first dimension of the N-dimensional data structure.

In some embodiments, the data circuit is arranged to separate/differentiate a slice from the further slice along a second dimension of the N-dimensional data structure.

According to an optional feature of the invention, the circuit is arranged to adapt the mapping of the samples of the segment into the slice in dependence on a size of the N-dimensional data structure in the first dimension.

According to an optional feature of the invention, adapting the mapping includes at least one of: - resampling the samples of the segment to generate a number of samples matching a number of elements in the slice; - mapping only a subset of the samples of the segment into the slice; or - mapping the samples of the segment into a subset of elements of the slice.

In some embodiments, the circuit is arranged to map data samples of a first time interval of the biomedical waveform time domain signal into a slice of elements of a first dimension of the N-dimensional data structure.

In some embodiments, the circuit is arranged to adapt the mapping of the samples of the first time interval into the slice in dependence on a size of the N-dimensional data structure in the first dimension.

In some embodiments, the mapping includes at least one of: - resampling the samples of the first time interval to generate a number of samples matching a number of elements in the slice; - mapping only a subset of the samples of the first time interval into the slice; or - mapping the samples of the first time interval into a subset of elements of the slice.

According to an optional feature of the invention, the adapter is arranged to determine the segment in dependence on the property of the biomedical waveform time domain signal.

In some embodiments, the adapter is arranged to determine a duration and/or size of the segment in dependence on a periodicity property of the biomedical waveform time domain signal.

According to an optional feature of the invention, N=2 and the N-dimensional transform coding is an image transform coding.

In some embodiments, the image transform coding includes a JPEG2000 coding.

According to an optional feature of the invention, the receiver is arranged to receive a further (second) biomedical waveform time domain signal and the circuit is arranged to map samples of the second biomedical waveform time domain signal into the N-dimensional data structure.

In some embodiments, the adapter is arranged to generate compressed data for a plurality of different values of at least one parameter of the arranging of samples of the biomedical waveform time domain signal into the N-dimensional data structure, to determine a quality measure for the compressed data for each of the plurality of different values; to select a first value of the plurality of different values dependent on the quality measure, and to adapt the arranging (mapping) of samples of the biomedical waveform time domain signal into the N-dimensional data structure to employ the first value.

In some embodiments, the adapter is arranged to analyze the biomedical waveform time domain signal to determine a first property of the biomedical waveform time domain signal and to adapt the arranging (mapping) of samples of the biomedical waveform time domain signal into the N-dimensional data structure dependent on the first property.

According to an optional feature of the invention, the adapter is arranged to apply a continuous wavelet transform (CWT), and in particular, a fast continuous wavelet transform (fCWT), to the biomedical waveform time domain signal to generate a set of continuous wavelet transform coefficients, and to determine the property from the set of continuous wavelet transform coefficients.

In some embodiments, the property may be a time-frequency dependent periodicity.

In some embodiments, the continuous wavelet transform may be a fast continuous wavelet transform. In some embodiments, the (fast) continuous wavelet transform may be a discrete wavelet transform (DWT). In some embodiments, the continuous wavelet transform may be a fast discrete wavelet transform (fDWT).

According to an optional feature of the invention, the N-dimensional transform coding comprises a wavelet transform and the adapter is arranged to adapt a property of the wavelet transform in dependence on the property.

In many embodiments, the property of the wavelet transform may be a mother and/or father wavelet.

According to an optional feature of the invention, the N-dimensional transform coding is a (typically discrete) wavelet transform coding using an N-dimensional (typically discrete) wavelet (transform).

According to an optional feature of the invention, the data signal generator is arranged to include an indication of the adaptation in the data signal.

According to another aspect of the invention, there is provided an apparatus for generating a reconstructed biomedical waveform time domain signal, the apparatus comprising: a receiver arranged to receive a data signal from a remote source, the data signal comprising compressed data representing a biomedical waveform time domain signal, the compressed data comprising a representation of an N-dimensional data structure; a decompression circuit arranged to determine the N-dimensional data structure from the compressed data by applying a decompression process to the compressed data, the compression process including an N-dimensional transform decoding; a circuit arranged to generate the reconstructed biomedical waveform time domain signal including arranging/mapping) data of the N-dimensional data structure into samples of the reconstructed biomedical waveform time domain signal; and an adapter arranged to adapt the arranging/mapping data of the N-dimensional data structure into samples of the reconstructed biomedical waveform time domain signal depending on an indication comprised in the data signal, the indication being indicative of a property of an arranging/mapping by the remote source of samples of the biomedical waveform time domain signal into the N-dimensional data structure.

According to another aspect of the invention, there is provided a method of generating a data signal representing a biomedical waveform time domain signal, the method comprising: receiving the biomedical waveform time domain signal; arranging samples of the biomedical waveform time domain signal into an N-dimensional data structure, N being no less than two; generating compressed data representing the biomedical waveform time domain signal by applying a compression process to the N-dimensional data structure, the compression process including an N-dimensional transform coding; generating the data signal to include the compressed data; and adapting the arranging of samples of the biomedical waveform time domain signal into the N-dimensional data structure dependent on a property of the biomedical waveform time domain signal.

According to another aspect of the invention, there is provided a method of generating a reconstructed biomedical waveform time domain signal, the method comprising: receiving a data signal from a remote source, the data signal comprising compressed data representing a biomedical waveform time domain signal, the compressed data comprising a representation of an N-dimensional data structure; determining the N-dimensional data structure from the compressed data by applying a decompression process to the compressed data, the compression process including an N-dimensional transform decoding; generating the reconstructed biomedical waveform time domain signal including arranging/mapping) data of the N-dimensional data structure into samples of the reconstructed biomedical waveform time domain signal; and adapting the arranging/mapping data of the N-dimensional data structure into samples of the reconstructed biomedical waveform time domain signal depending on an indication comprised in the data signal, the indication being indicative of a property of an arranging/mapping by the remote source of samples of the biomedical waveform time domain signal into the N-dimensional data structure.

These and other aspects, features and advantages of the invention will be apparent from and elucidated with reference to the embodiment(s) described hereinafter.

### BRIEF DESCRIPTION OF THE DRAWINGS

Embodiments of the invention will be described, by way of example only, with reference to the drawings, in which
FIG. 1 illustrates some elements of an example of a system for processing biomedical waveform signals;
FIG. 2 illustrates some elements of an example of an apparatus for generating a data signal comprising compressed data representing a biomedical waveform signal in accordance with some embodiments of the invention;
FIG. 3 illustrates some elements of an example of an apparatus for generating a reconstructed biomedical waveform signal from a data signal comprising compressed data representing the biomedical waveform signal in accordance with some embodiments of the invention;
FIG. 4 illustrates an example of a wavelet transform structure;
FIG. 5 illustrates an example of a frequency span for different levels of a wavelet transform structure;
FIG. 6 illustrates an example of a time-frequency tiling for a wavelet transform structure;
FIG. 7 illustrates some elements of an example of a system for processing biomedical waveform signals;
FIG. 8 illustrates some elements of a possible arrangement of a processor for implementing elements of an apparatus for processing/reconstructing biomedical waveform signals in accordance with some embodiments of the invention.

### DETAILED DESCRIPTION OF SOME EMBODIMENTS OF THE INVENTION

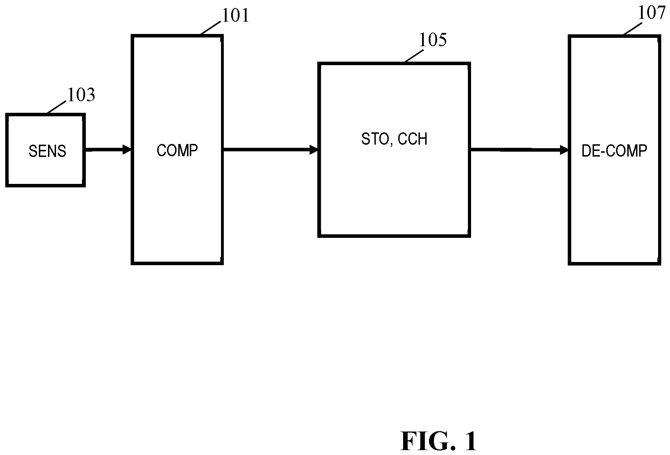

FIG. 1 illustrates an example of a system for generating a data signal comprising compressed data representing a biomedical waveform signal and for reconstructing the biomedical waveform signal from the compressed data.

The system comprises a compression circuit or device 101 which is arranged to receive a biomedical waveform signal from a suitable biomedical sensor.

In many embodiments, the biomedical waveform signal may be an electrocardiography (ECG) signal which specifically may be provided from a sensor comprising a plurality of electrodes that are positioned on the subject's body to pick up electrical signals relating to the heart operation.

In some embodiments, the biomedical waveform signal may be an electroencephalography (EEG) signal which specifically may be provided from a sensor comprising a number of electrodes that are positioned on the subject's head to pick up electrical signals relating to brain activity.

In some embodiments, the biomedical waveform signal may be an electromyography (EMG) signal which specifically may be provided from a sensor comprising a plurality of electrodes that are positioned on the subject's body to pick up electrical signals relating to muscle movement of the person.

In some embodiments, the biomedical waveform signal may be a photoplethysmography (PPG) signal which specifically may be provided from a sensor comprising a photodetector arranged to measure the intensity of reflected light from tissue and skin of a person. Such a detection may for example provide a measurement of blood volume/flow changes or heart rate.

The biomedical waveform signal is specifically a time domain signal with a time domain waveform that reflects the changes in the signal from the sensor as a function of time.

The compression device 101 proceeds to process the biomedical waveform signal to generate a data signal which includes compressed data representing the biomedical waveform signal.

In many embodiments, the compression device 101 may receive a plurality of biomedical waveform signals which may each be encoded/compressed as described, i.e., compressed data may be generated representing each of the biomedical waveform signals.

The data signal may then be provided to an intermediate circuit/function 105 from which it is provided to a decompressor circuit or device 107 which is arranged to generate a reconstructed biomedical waveform signal from the received data signal, and specifically from the compressed data representing the biomedical waveform signal.

The intermediate circuit/function 105 may for example be a distribution or communication medium/channel/network. For example, the data signal may be communicated from the compression device 101 to the decompressor device 107 via the Internet or another communication network. As another example, the intermediate circuit/function 105 may be a store or memory. For example, in some embodiments, the compression device 101 and the decompressor device 107 may be implemented as part of the same device with the biomedical waveform signal being compressed for local storage following the capture and decompressed when later being extracted from the local storage. In some embodiments, the intermediate circuit/function 105 may be suitable both for storage and distribution. For example, a captured biomedical waveform signal may be stored on a memory card, such as an SD card, or a USB drive or any other removable storage medium which can then be removed and provided to a different device where the data signal is extracted from the memory card and decompressed to provide a reconstructed biomedical waveform signal.

In order to reduce the storage and/or communication demands for the intermediate circuit/function 105, it is desired that the data size/rate of the data signal is as small as possible, yet that it still allows a sufficiently accurate reconstruction of the biomedical waveform signal. Accordingly, it is desired that the compression and decompression processes are as efficient and accurate as possible. As such it is desired that the generated data signal provides an efficient data representation that supports such efficient compression and decompression operations. Further, in order to facilitate the implementation and operation, it is desired that the data signal allows an efficient representation and a suitable selection of data and parameters. Accordingly, a suitable selection and structure of data for the data signal is desired to improve the overall performance.

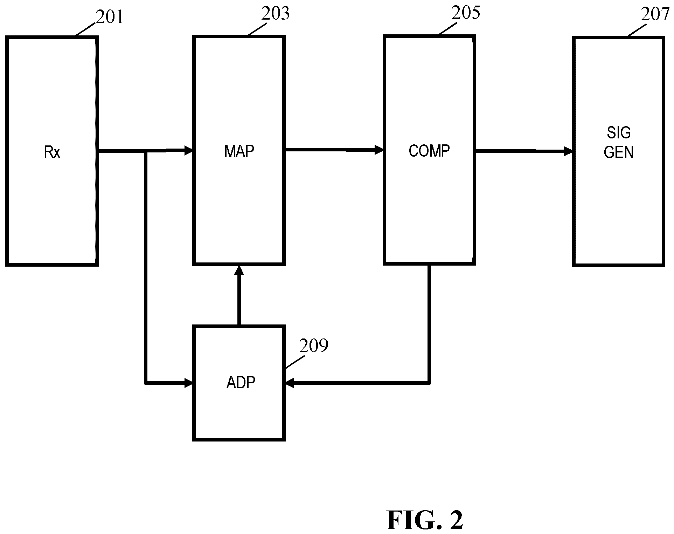

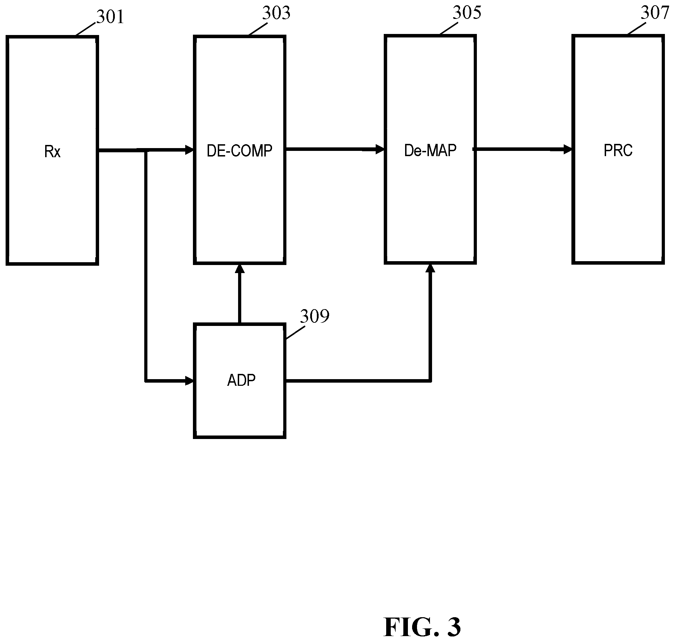

FIG. 2 illustrates an example of some elements of the compression device 101 of FIG. 1 and FIG. 3 illustrates an example of some elements of the decompressor device 107 of FIG. 1.

The compression device 101 comprises a receiver 201 which receives the biomedical waveform signal from the sensor 103. The receiver 201 is coupled to a mapping circuit 203, which as will be described in more detail later, arranges samples of the biomedical waveform time domain signal into a higher dimensional data structure. The mapping circuit 203 is coupled to a compressor 205 which is arranged to perform a compression process to generate compressed data from the biomedical waveform signal. The compression is specifically performed on the higher dimensional data structure as will be described in more detail later. The compression may be an encoding/compression which results in a representation of the biomedical waveform signal which has a reduced data rate/size relative to the biomedical waveform signal received from the sensor 103. As will be described in more detail later, the compression may in some cases be a lossy compression and may in some cases be a lossless compression.

The compressor 205 is coupled to a data signal generator 207 which is arranged to receive the compressed data for the biomedical waveform signal and to generate a data signal that includes the compressed data. As will be described further later, the data signal may also include other data, such as data describing other signals or other auxiliary data.

The data signal may then be stored/retrieved and/or transmitted/communicated in order to be provided to the decompressor device 107. The decompressor device 107 accordingly comprises a receiver 301 which receives the data signal. The receiver 301 is coupled to a decompressor 303 and is arranged to extract the compressed data from the received data signal and provide it to the decompressor 303.

The decompressor 303 is arranged to perform a decompression process on the compressed data to generate a reconstructed biomedical waveform signal. The decompression process ideally reconstructs the higher dimensional data structure that was compressed by the compressor 205. The decompressor 303 is coupled to a de-mapping circuit 305 which is arranged to map the data values of the reconstructed higher dimensional data structure into a reconstructed biomedical waveform time domain signal, and thus it maps from the higher dimensional data structure into a one-dimensional time domain signal.

If the compression/decompression processes are ideal and lossless (and no data errors are introduced by the storage/communication), the reconstructed biomedical waveform signal will in principle be identical to the biomedical waveform signal input to the compressor 205. However, in many cases, in order to provide a sufficient reduction in the data rate by the compression approach and/or to maintain low complexity/resource usage, the reconstructed biomedical waveform signal may often deviate from the original biomedical waveform signal but will typically be a sufficiently close approximation thereto such that it allows for further application, such as subjective clinical evaluation for detecting anomalies or feature extraction processing, e.g., deriving the heart-rate or breathing-rate from an PPG signal.

The decompressor 303 is coupled to a processor 307 which is fed the reconstructed biomedical waveform signal, and which is arranged to perform a processing of the reconstructed biomedical waveform signal. Many different approaches and techniques based on a biomedical waveform signal are known and the use/processing of the biomedical waveform signal depends on the individual embodiments and indeed on the specific nature of the biomedical waveform signal, and in particular on the type of the biomedical waveform signal. For example, in many embodiments, the biomedical waveform signal may be processed such that the waveform can be displayed on a suitable display. In other scenarios, the reconstructed biomedical waveform signal may be analyzed to extract statistical properties, may be compared to other signals (specifically other biomedical waveform signals), etc.

In the approach, the compressor 205 and decompressor 303 are arranged to employ particular advantageous compression/decompression processing which for many biomedical waveform time domain signals may result in a particularly advantageous trade-off between data rate of the compressed data and the data signal and the accuracy and quality of the reconstructed biomedical waveform time domain signal.

The mapping circuit 203 arranges/maps samples of the biomedical waveform time domain signal into an N-dimensional data structure where N is no less than two, i.e., N≥2. Each element/value of the N-dimensional data structure may accordingly be identified by N indices into the N-dimensional data structure.

The mapping of the samples of the biomedical waveform time domain signal may for example be by filling up the N-dimensional data structure sequentially one dimension/index at a time. For example, samples may be added one at a time by increasing one index until the index reaches the end (e.g, the maximum) index value. It may then increment a second dimension index and then repeat the process. When the next dimension index reaches the end/maximum value, a third index dimension may be incremented (stepped) and the process repeated again, and so on and on, e.. until all the data has been mapped to the N-dimensional data structure.

Although it will be appreciated that dimensions higher than two may be used in many embodiments, the use of a 2-dimensional data structure, i.e. N=2, may be particularly advantageous in many scenarios and for many biomedical waveform time domain signals. Thus, in some embodiments, the N-dimensional data structure may be a two-dimensional arrangement such as specifically a bidimensional array of data values. The N-dimensional data structure may thus be a structure of rows and columns. In such cases, the mapping circuit 203 may be arranged to divide the biomedical waveform time domain signal into a plurality of time intervals and then populate each row (or alternatively column) using samples from one time interval with the column (or alternative row) index being incremented for each time interval.

Specifically, in many embodiments, the mapping circuit 203 may be arranged to map data samples of a given time interval of the biomedical waveform time domain signal into a slice of elements of a given dimension of the N-dimensional data structure. In many embodiments, the biomedical waveform time domain signal may be divided into time intervals with the samples of each time interval being mapped into different slices of the N-dimensional data structure. The slices may be in the same dimension, e.g., the slices may be rows (or alternatively columns) of a 2-dimensional data structure. In many embodiments, the slices may have a size/length equal to the size of the N-dimensional data structure in the dimension of the slice.

In some embodiments, the mapping circuit 203 may be arranged to map data samples of a given time interval of the biomedical waveform time domain signal into a slice of elements of a given dimension of the N-dimensional data structure with a random arrangement; e.g., the mapping may follow a random selection of the N-dimensional data structure element to which to copy the mapped data. This selection can be easily reversed e.g. by the arrangement being a pseudo-random process indexed by the initial seed. The initial seed can be selected from another random process for different waveform time domain signals.

In some embodiments, the number of dimensions of the N-dimensional data structure can additionally or alternatively be selected with the same random algorithm.

The N-dimensional data structure is fed to the compressor 205 which applies a compression process to the N-dimensional data structure where the compression process includes performing an N-dimensional transform coding. The N-dimensional transform coding is applied to the N-dimensional data structure (possibly after some modification such as a scaling or filtering of the data values of the structure).

Different N-dimensional transform coding algorithms may be used in different embodiments. For example, in the example of a 2-dimensional data structure, the compressor 205 may apply a spatial fourier transform to the 2-dimensional data structure to generate coefficients that may be suitable for efficient encoding. As another example, the N-dimensional transform coding may be Discrete Fourier Transform (DFT), a Fast Fourier Transform (FFT), a Short Time (Windowed) Fourier Transform (STFT or WFT), a (Modified) Discrete Cosine Transform ((M)DCT) or (Modified) Discrete Sine Transform ((M)DST) which are commonly used in 2D or 3D image coding, etc.

The decompressor 303 may perform the inverse N-dimensional transform to reconstruct the N-dimensional data structure and the de-mapping circuit 305 may proceed to perform the inverse mapping operation of the mapper 205. For example, it may proceed to extract rows (or alternatively columns) sequentially and generate a time interval of the reconstructed biomedical waveform time domain signal from each row (column).

In many embodiments, the de-mapping circuit 305 may map elements of different slices in the same dimension into different time intervals of the reconstructed biomedical waveform time domain signal.

In some embodiments the de-mapping circuit 305 may reverse the pseudo-random transform from the N-dimensional data to the reconstructed biomedical waveform time domain signal.

In many embodiments, the N-dimensional transform coding may be a (N-dimensional) wavelet transform coding. The wavelet transform may e.g. be one using an isotropic and/or polyharmonic N-dimensional wavelet. In particular, for the 2D case, the wavelet transform may use 2-dimensional (e.g. dyadic) wavelet/wavelet analysis. In many embodiments, the wavelet transform may specifically be a discrete wavelet transform.

In many embodiments, the compression process may specifically employ a wavelet transform and the compression may be a wavelet compression algorithm/process. Correspondingly, the decompression may perform a wavelet decompression algorithm. Thus, in many embodiments, the compressed data includes wavelet transform coefficient data generated by wavelet compression process and the decompression may apply a wavelet decompression algorithm to the wavelet transform coefficient data. The Inventors have realized that a wavelet compression/decompression approach as described is particularly suitable and advantageous for compression and decompression of biomedical waveform signals, and in particularly for compression and decompression of an electrocardiography (ECG), electroencephalography (EEG), electromyography (EMG), or photoplethysmography (PPG) signal, (and indeed for a number of other biomedical signals).

Indeed, wavelet transforms, and in particular the Discrete Wavelet Transform (DWT), are well suited for processing of signals of physical nature (time domain, discrete type) that can be found in real-life.

A DWT may (e.g. initially) split the time-domain signal into a low frequency band using a filter g0[n] and a high frequency band using a filter h0[n]. The low-band filter g0[n] and the high-band filter h0[n] are typically complementary filters such that these constitute a critically sampled filter-bank. As a result, the outputs of the analysis filters g0[n] and h0[n] may be critically sampled (decimated by a factor 2) such that the sampling rate at the input time-domain signal x[n] is the same as the (combined) sampling rate at the outputs of the analysis filters g0[n] and h0[n]. There are thus no additional samples (data) generated at the output of the analysis filter bank. Using a complementary synthesis structure with filters g1[n] and h1[n], perfect or near perfect reconstruction may be obtained. In the case of near-perfect reconstruction, the error after the reconstruction falls below a certain threshold, typically the selected resolution of the time-domain representation of the biomedical signals, such as 16 or 24 bits resolution, but also other thresholds may be used, such as frequency dependent thresholds to provide for a frequency dependent reconstruction error. The choice of the wavelet filters provides a degree of freedom in the design of the DWT. Critical sampling of filter banks as well as (near) perfect reconstruction is well known from the audio coding domain using critically sampled filter banks such as Quadrature Modulate Filter (QMF) or Modulated Discrete Cosine Transform (MDCT) filter banks. Typically, the filter banks used in the audio coding domain use much more than 2 filters, for example 32 or 64.

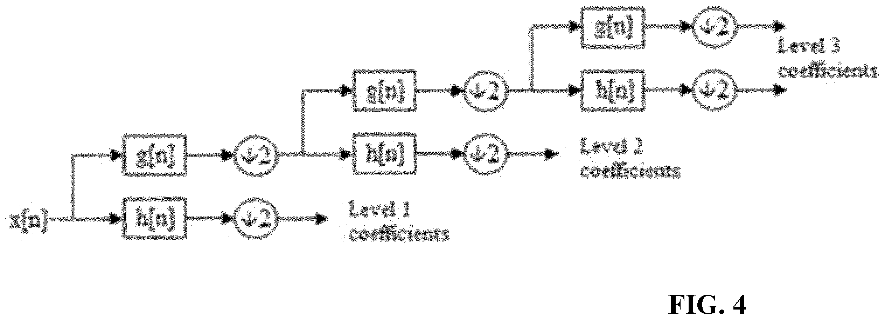

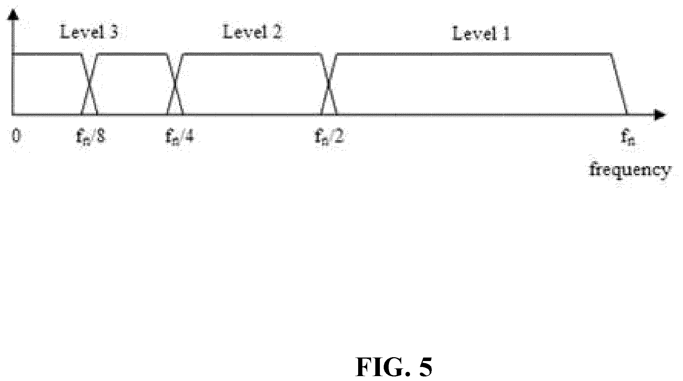

As illustrated in FIG. 4, in a DWT, for each subsequent level, only the low band is typically further split using the same analysis filter g0[n] (in the figure indicated simply by the reference g[n] and with h0[n] being indicated by h[n]), resulting in a further split of the low frequency band until eventually a filter tree up to a level J is obtained (see Fig. 5). In the context of wavelet filterbanks, the LF (low frequency) portions are denoted as Approximation coefficients'. The HF (high frequency) portions are denoted as 'Detail coefficients'. Detail coefficients can be seen as what is missing from the approximation at level J to get to the approximation at level J-1.

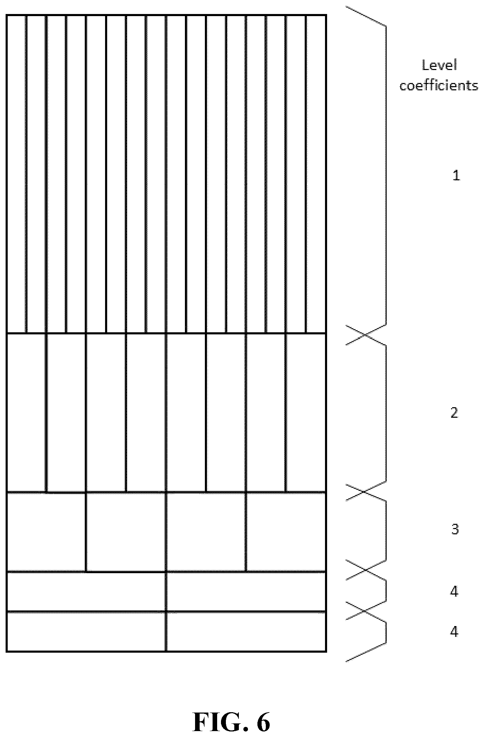

When applying a DWT onto a time domain signal, it produces a set of coefficients that represent specific time-frequency tiles as illustrated in FIG. 6, with time on the horizontal axis and frequency on the vertical axis, where each block represents a specific time-frequency interval, i.e., an area bounded by a specific time and frequency interval. In practice, the 'bounding' is not hard because of the non-perfect frequency resolution of practical filters, i.e., in practical implementations, filters are of finite length and therefore brick-wall filters are not feasible. Low frequency coefficients have a high frequency resolution but a low time resolution. On the other hand, high-frequency coefficients have a low frequency resolution but high time resolution (see Heisenberg boxes/rectangles in time-frequency representations). This combination of frequency and time resolution is compared to match well to physical processes. In comparison, general subband- or frequency-transform filter banks typically employ a uniform division of the time/frequency plane.

The DWT may be applied to subsequent segments of a signal so that a block-based processing is enabled. This is particularly useful for use cases that require random access into the compressed representation (bitstream) representing the biomedical signal.

Typically, the compressed data may be encoded and decoded as part of being included in the data signal and being extracted therefrom. Thus, the compressed data of the data signal may be encoded compressed data.

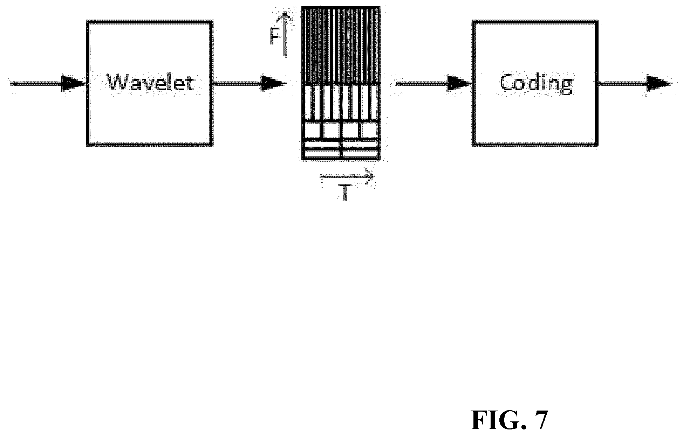

For example, as illustrated in FIG. 7 an approach based on wavelet transforms. In the example, the generated wavelet coefficients are further compressed by an encoding that includes a data compression. Typically, this encoding may comprise different techniques including:
• Removal of redundancy - lossless coding techniques such as prediction, entropy coding, ...
· Removal of irrelevancy - lossy coding techniques such as quantization, noise substitution, ...

Redundancy removal may preserve the perfect reconstruction of the input signal. After irrelevancy removal, perfect reconstruction is generally no longer feasible.

Exploiting redundancy may for example include encoding parts of the biomedical waveform signal/some wavelet coefficients relative to other parts of the biomedical waveform signal/wavelet coefficients. Principally, the removal of irrelevance is often obtained by e.g., simple thresholding (quantization) (i.e., putting to zero coefficients less than a given threshold) thanks to the relevant property of the wavelet transform of concentrating the most relevant information of the original signal resides in the biggest (by absolute magnitude) coefficients.

For entropy coding various techniques and methods are available, such as Huffman coding or Arithmetic coding. Of importance to the global efficiency of the entropy compression are also techniques that try to separate the signal into well behaved (i.e., uniform) components like splitting the sign and mantissa, delta coding etc., of the wavelet coefficients.

In the approach, the arrangement of samples into the N-dimensional data structure is not a fixed predetermined arrangement or mapping but is an adaptive arrangement/mapping.

Accordingly, the compression device 101 comprises an adapter 209 which is arranged to adapt the arranging/mapping of samples of the biomedical waveform time domain signal into the N-dimensional data structure dependent on a property of the biomedical waveform time domain signal. The adapter 209 is coupled to the receiver 201 and receives the biomedical waveform time domain signal from which a property may be determined. It is further coupled to the mapping circuit 203 which adapts the mapping to the N-dimensional data structure based on the determined property. The property may be a composite/combined property comprising different values representing different characteristics of the signal.

In many embodiments, the property of the biomedical waveform time domain signal may be a periodicity or repetition property for the biomedical waveform time domain signal.

Many, if not most, biomedical waveform time domain signals used in practice have an element of repetition or periodicity. For example, measurements that have a dependency on the pulse or heart movement will inherently include a repeating, and typically substantially periodic, component.

As a specific example, an ECG will generate a biomedical waveform time domain signal that is periodic with a rate equal to the heart rate. Such a periodicity may for example be detected by detecting e.g., the time between peaks, the time offset for a maximum autocorrelation value etc.

As another example, a PPG biomedical waveform time domain signal (specifically representing a photoplethysmogram) will have a periodicity matching the pulse and this can often easily be detected using e.g., peak detection.

In some cases, a biomedical waveform time domain signal may have different periodic properties/components. For example, a photoplethysmogram may have a periodicity corresponding both to a pulse rate and a breathing rate. In some cases, the periodicity property may reflect multiple periodic components, and in many embodiments a periodicity property reflecting a single periodicity component may be determined.

The periodicity component may in many embodiments be determined to reflect a period time (or equivalently a frequency) and/or a timing (offset) indication, such as an indication of a timing of a particular event/characteristic of the periodic component (such as an onset time, end time, peak time etc.).

The mapping circuit 203 may then proceed to adapt the arrangement of the samples of the biomedical waveform time domain signal into the N-dimensional data structure dependent on such a periodic property. For example, the mapping circuit 203 may be arranged to segment the biomedical waveform time domain signal into time intervals with the samples of each time interval being fitted into one slice (of maximum size) of a given dimension (such as a row of a 2-dimensional data structure). The mapping circuit 203 may then be arranged to adapt the properties of the N-dimensional data structure (such as the size in the dimension of the slice) and/or the segmentation of the biomedical waveform time domain signal, and/or the fitting of the samples of a time interval into a slice in dependence on the periodic property.

As a specific example, a suitable start time and period time for each cycle of an ECG may be determined and the segmentation and the size of a 2-dimensional data structure may be determined accordingly. For example, the segments may be set to have a starting time matching a start time of a given heart cycle and with a duration equal to the determined period time. The resulting number of samples in each segment may then be determined and the 2-dimensional data structure may be set to have a size where the size of one dimension includes a number of elements that match the number of samples in a segment. The mapping circuit 203 may then proceed to map the samples of each segment into a single slice along this dimension, e.g., in the specific example with each row comprising a number of elements matching the number of samples in the segments. Thus, in the example, each slice/row comprises one segment of the biomedical waveform time domain signal corresponding to one period/one cycle of the heart rate. In many embodiments, such as the previous example, the adaptation of the arrangement of the samples of the biomedical waveform time domain signal into the N-dimensional data structure is thus performed by adapting a segmentation of the biomedical waveform time domain signal and adapting a size of the N-dimensional data structure (but it will be appreciated that in some embodiments only one of these adaptations may be performed). In many embodiments, the segmentation and/or a size of the N-dimensional data structure may be adapted such that the number of samples in a segment matches a (maximum) number of elements in a one-dimensional slice of the N-dimensional data structure (for at least one dimension).

In other embodiments, other properties of the biomedical waveform time domain signal may be considered, such as for example a type of the biomedical waveform time domain signal (e.g., whether it is an analog or digital signal or whether it is an whether it is an ECG, EEG, EMG, or PPG signal ), a sampling rate, a duration, a frequency composition, etc.

For example if the type of the biomedical waveform time domain signal differs in the way of the number of components (channels), or in the way of different lengths (sampling rate, or shorter acquisition on some channels than in others etc.) in the different channels the mapping circuit 203 will handle such situations with proper data techniques to deal with the missing data values problem such, but not limited to, data dropping (reduce to the shortest) and/or data padding (augment to the longest), mean or median imputation, random sample imputation, multivariate multiple imputation, regression imputation, maximum likehood estimation etc. In this way the mapping by the circuit 203 to the N-dimensional space is reduced to the previous examples.

In another example if the type of the biomedical waveform time domain signal differs in the way of the signal in different channels one is analog and the other digital the mapper circuit 203 will resort to make a uniform selection of the signal converting to the same analog or digital format depending, for example, on the signal with the lowest sampling frequency or considering signal processing properties typical of the field and commonly known to the expert skilled in the art.

In many embodiments, the data signal generator is arranged to include an indication of the adaptation in the data signal. The data signal generator 207 may specifically include an adaptation indication which indicates the value of a variable parameter of the mapping process which has resulted from the adaptation. For example, the data signal generator 207 may include an adaptation indication which indicates a segment size, starting time, and/or an indication of a size of (at least one dimension of) the N-dimensional data structure.

The decompressor device 107 may be arranged to adapt the decompression operation dependent on the adaptation indication. Accordingly, the decompressor device 107 comprises a demapping adapter 309 which is coupled to the de-mapping circuit 305 and the receiver 301. The demapping adapter 309 may be fed the adaptation indication and proceed to adapt the arranging/mapping of the received reconstructed N-dimensional data structure into the samples of the biomedical waveform time domain signal and/or the decompression/ reconstruction of the N-dimensional data structure in dependence on the adaptation indication. For example, it may proceed to adapt the size of the N-dimensional data structure to match the one used at the encoder and indicated in the adaptation indication.

As mentioned above, the adapter 207 may in many embodiments adapt a property of the N-dimensional data structure in dependence on a property of the biomedical waveform time domain signal, and the demapping adapter 309 may in response to the adaptation indication adapt the reconstruction of the biomedical waveform time domain signal to match the processing of the compression device 101.

As mentioned, in many embodiments, the adapted property of the N-dimensional data structure may be a size of the N-dimensional data structure for at least one dimension. In other embodiments, other properties may alternatively or additionally be adapted. For example, the overall size of the N-dimensional data structure may be adapted depending on the biomedical waveform signal.

As an example, the overall size of the N-dimensional data structure may be set dependent on a property of the biomedical waveform time domain signal, such as for example a duration of the biomedical waveform time domain signal and/or e.g., a frequency distribution thereof. For example, the total size of the N-dimensional data structure may be set to be sufficiently large to include all samples of the biomedical waveform time domain signal. As another example, if the biomedical waveform time domain signal comprises significant high frequency components, the overall size may be set to include all samples of the biomedical waveform time domain signal. However, if the biomedical waveform time domain signal does not have any (or only insignificant) high frequency components, the overall size may be reduced. For example, in such a case, only e.g., every other sample of the biomedical waveform time domain signal may be mapped to the N-dimensional data structure, and the size of the structure may be halved (corresponding to a decimation by two being applied in a case where this still allows the Nyquist requirement to be met).

In some cases, the dimension of the N-dimensional data structure may be adapted, i.e., N may be dependent on the biomedical waveform time domain signal. For example, the dimension of the N-dimensional data structure may be dependent on the type of the biomedical waveform time domain signal. For example, a higher dimensional N-dimensional data structure may be used for an EEG signal than for an ECG signal.

As another example, the measured or pre-set deviation in the number of samples between peaks in an ECG signal may determine the dimension of the N-dimensional data structure.

As another example, the dimension of the N-dimensional data structure can be chosen to minimize a metric in the N-dimensional space, like, for example, the volume of the represented data. In a more specific context, the dimension of the N-dimensional data structure can be chosen to minimize or maximize the entropy of the data along one or a subset or all the dimensions of the selected space.

As another example, the dimension of the N-dimensional data structure can be chosen randomly from a set of limits dictated from the dimension of the biomedical waveform time domain signal. In this case it can be implemented algorithmically as an optimization problem looking for a minimum of some energy function defined by the objective of the minimization together with some numerical constraints (e.g., minimum and maximum number of dimensions etc.)

Another example is to choose the dimension of the N-dimensional data structure as a function of the selected mother wavelet so as to optimize its performance on the given biomedical waveform time domain signal.

It must be noted that some, if not all, of the given examples may also indicate that the compression algorithm 101 could/may potentially influence the inner workings (e.g., sampling rate etc.) of the sensor 103 acquiring the biomedical waveform time domain signal, i.e. that the apparatus may be arranged to adapt a property of the sensor 103/capturing of the biomedical waveform time domain signal in dependence on a property of the N-dimensional data structure.

As described above, in some embodiments the arrangement of the samples of the biomedical waveform time domain signal into the N-dimensional data structure may include adapting the N-dimensional data structure and/or a segmentation of the biomedical waveform signal into segments that are then mapped to slices of the N-dimensional data structure.

In many embodiments, the mapping circuit 203 may be arranged to adapt the mapping of samples from a given segment into a given slice dependent on the size of the N-dimensional data structure in the dimension of the slice. The mapping circuit 203 may be arranged to adapt the mapping of samples from a given segment into a given slice dependent on the size of the slice.

For example, in some embodiments the size of the N-dimensional data structure and/or the slice may be determined such that they do not match exactly, and there may be differences between the number of samples in the segments and the number of elements in the slices. This may for example be the case when one or both of these are fixed in size but with different sizes, or more typically if one of the sizes is fixed/predetermined but the other is dynamically adapted based on the biomedical waveform time domain signal. A mismatch may in particular occur in situations where a fixed N-dimensional data structure is employed, such as e.g., where a given size of a 2-dimensional data structure is predefined, while the duration, and thus the number of samples, of the segments may vary. As a specific example, the N-dimensional data structure may be fixed while the segments are adapted to e.g., match the heart rate captured by the biomedical waveform time domain signal.

In such cases, the mapping of samples from a segment of the biomedical waveform time domain signal into a slice of the N-dimensional data structure may be adapted to compensate for the differences. Thus, effectively, time domain adaptation may be performed to map a given segment size/segment time interval to match the number of elements of the slice. Essentially, the mapping may be adapted to "fit" the segments into the slices. Such a fitting may in many embodiments include a potential "stretching" or "expanding" (also sometimes referred to as "morphing" or "mesh morphing") of the segment to fit the slice and/or a potential "compression" of the segment to fit the slice.

For example, that mapping/adaptation may be such as to make the period of each beat constant by a nearly reversible transformation, in which the original periods can be restored without any loss. Such a process of normalization of each isolated heart beat can be obtained, for example, by multi-rate techniques; i.e., sampling rate changes by different fractional factors for different heart beat cycles. This process may result in a conversion of the heart beats of different lengths (periods) into heart beats of constant length (period) essentially eliminating the effects of heart beat variability. The length of the normalizing heart beat period can be selected, among other choices, from the maximum length of any cardiac heartbeat and the sampling frequency of the original signal. This selection should typically obey the Nyquist criterion so that the selected length assures that the sampling rate is higher than the original one so that no distortion of the acquired heart electric signal is created. Some information like the mean value of the original heartbeat period may be encoded in the compressed signal to be used by the decoder to recover the original signal. This normalization of the heart period can, for example, use the processes of interpolation and downsampling by an appropriate factor for each of the original signal heartbeat periods. A polyphase interpolation filter can be used for an efficient interpolation process.

In some embodiments, the mapping circuit 203 may be arranged to resample the samples of the segment to generate a number of samples that match the number of elements in the slice. Such a resampling may include an upsampling and/or a downsampling as appropriate. In particular, such a resampling may modify the representation of the biomedical waveform time domain signal in the segment from the number of samples in the segment to the number of elements in the slice. The resampling may include decimation, interpolation etc., as is well known in the art.

In some embodiments, the mapping circuit 203 may be arranged to map only a subset of the samples of the segment into the slice. For example, if the number of samples in the segment is higher than the number of elements in the slice, the mapping circuit 203 may be arranged to only map a number of the elements in the segment that can fit in the slice. The remaining samples may simply be discarded. The selection of the samples being mapped may depend on the individual embodiment. In many cases, the appropriate number of samples at the beginning and/or end of the segment may simply be discarded (thus the segment may effectively be truncated). In some embodiments, a dynamic selection may be performed such as for example based on a detection of particularly significant parts of the segment, such as e.g., those representing the main variation/ core QRS complex of an ECG cycle.

In some embodiments, the mapping circuit 203 may be arranged to map the samples of the segment into a subset of elements of the slice. For example, if the number of samples is smaller than the number of elements in the slice, the mapping circuit 203 may directly map the samples to a corresponding number of sequential elements of the slice. The remaining elements may then be populated e.g., with predetermined values (such as simply setting the values of such elements to zero or e.g. to values determined by an interpolation process).

As mentioned, the N-dimensional data structure may in many embodiments particularly be a 2-dimensional data structure. This may provide a particularly advantageous operation in many embodiments and is particularly suitable for representing properties of a biomedical waveform time domain signal, including typically periodic properties and both time and frequency characteristics. Further, a 2-dimensional data structure is particularly suitable for compression algorithms that provide efficient results for biomedical waveform time domain signal, and is particularly highly suitable for many types of wavelet transform compression/coding.

For a 2-dimensional data structure, the elements/data samples of the biomedical waveform time domain signal may be arranged as a two-dimensional array/matrix with all lines having the same size and all columns having the same size. The 2-dimensional data structure may be a matrix representation and thus may essentially be a 2-dimensional structure with a given aspect ratio.

In many embodiments, such a 2-dimensional data structure may then advantageously be compressed using a wavelet transform based on a 2-dimensional (mother) wavelet.

Further, in some embodiments, the encoding and compression of the 2-dimensional data structure may be performed using an image transform coding. For example, the 2-dimensional data structure may be fed to an image transform coding algorithm which may process the 2-dimensional data structure as if it were an image. The image processing process may generate a very efficient encoding of the 2-dimensional data structure. At the decompressor device 107, the inverse process may be performed, i.e., the image decompressor 303 may perform the inverse image compression to reconstruct the 2-dimensional data structure.

In many embodiments, the image transform coding/decoding may specifically include/be based on a JPEG2000 coding. Such an encoding/decoding process may provide a particularly advantageous operation and performance. The JPEG2000 encoding is based on a wavelet compression/encoding and the Inventors have realized that the approach of the JPEG2000 coding provides a very efficient encoding when used with a 2-dimensional data structure representing one or more biomedical waveform time domain signals. It furthermore allows re-use of some existing algorithms and systems thereby facilitating implementation and operation in many embodiments.

The previous description has focused on encoding and compression of a single biomedical waveform time domain signal. However, in many embodiments, the compression device 101 may be arranged to encode/compress one or more further signals and include compressed data for such further signals in the data signal.

In many embodiments, the further signal may be another biomedical waveform time domain signal and indeed, may be another signal measured as part of the same measurement. For example, in many embodiments, the data signal may include a plurality of biomedical waveform time domain signals that are all related to the same underlying biomedical measurement. For example, the data signal may include a biomedical waveform time domain signal for each electrode/sensor of a measurement that includes a plurality of such electrode/sensors. For example, for an ECG or EEG a plurality of electrodes is in contact with the test subject/patient and a plurality of signals are measured together. In many embodiments, the data signal may include all such signals from a measurement with each of these biomedical waveform time domain signals being compressed and represented by compressed data in the data signal. Such different biomedical waveform time domain signals may also often be referred to as different channels, and specifically as different channels of the biomedical measurement.

As an example, each captured biomedical waveform time domain signal of a measurement may individually be compressed as previously described for a single biomedical waveform time domain signal. The resulting compressed data for all biomedical waveform time domain signals may then be included in the data signal. However, in some embodiments, some cross signal/cross channel compression may be applied to provide a more efficient encoding of the biomedical waveform signals.

The decompressor device 107 may proceed to reconstruct all the biomedical waveform signals using the approach previously described. The data signal may be generated to include an indication of the signals, and specifically the biomedical waveform time domain signals, that are included in the data signal, and possibly also how they are encoded, such as e.g., whether they are encoded individually or jointly. The decompressor device 107 may then adapt the processing to reconstruct all the signals using complementary/inverse operations.

In some embodiments, the further signal may be a non-biomedical waveform signal. For example, the data signal may further include one or more accelerometer data, data relating to the environment, audio signals as well as e.g., diagnostically relevant sensors data/signals, such as e.g., sweating, skin conductivity etc. The data signal may in some cases include other signals that are convenient or practical to include in the data signal e.g., simply due to it providing potentially relevant information that e.g., may be useful for diagnostic purposes. Alternatively or additionally, the data signal may include some signals that are correlated with one or more biomedical waveform signals although not directly part of the same measurement. For example, an accelerometer positioned appropriately on a patient may generate a waveform that has common properties with the biomedical waveform signals. For example, an accelerometer positioned near the heart of a person may generate a signal that has the same and/or a related periodicity to that of biomedical waveform signals measured as part of an ECG measurement.

In many embodiments, one or more of the further signal(s) may accordingly be a measurement of a different modality than the biomedical waveform signal and may specifically be a different type of signal.

In some cases, the compression device 101 may be arranged to utilize such signals in the compression of the biomedical waveform signals and the decompressor device 107 may similarly be arranged to utilize the signals in the decompression to reconstruct the biomedical waveform signals.

In many embodiments, such further signal(s), whether biomedical waveform time domain signal(s) or non-biomedical waveform time domain signal(s), may be mapped into the same N-dimensional data structure as the first biomedical waveform time domain signal. The resulting combined N-dimensional data structure may then be compressed/encoded with this including the N-dimensional transform coding, and specifically may be encoded using an image compression coding for the 2-dimensional case.

In many embodiments, the mapping may be such that it exploits that the signals are correlated and have similar properties. For example, different channels/signals for a single measurement, such as e.g., an ECG or EGC measurement, will have the related periodicity. Similarly, an accelerometer positioned on the chest of a user will tend to exhibit the related periodicity (such as e.g. having a corresponding period time or frequency).

Such commonality may be used when mapping the different signals into a shared N-dimensional data structure. For example, in many cases, the same segmentation may be applied to all signals (specifically the segments for different signals are for the same time intervals). In some cases, a relative delay may be introduced to the signals as part of the segmentation in order to align the segments to the underlying physiological function and to compensate for differences in e.g. the time of propagation through the body. Thus, segments may be generated which in time match the same underlying physiological function. For example, for a given heart rate period, a segment may be generated for each of the multiple signals.

The mapping circuit 203 may then be arranged to map the samples of the segments into the shared N-dimensional data structure. Specifically, the segments of the different signals may then be entered into slices along one dimension of the N-dimensional data structure. For example, the segments of different signals may be interlaced into different slices in the N-dimensional data structure. As a specific example, for a 2-dimensional data structure, the segments of the different signals may be mapped to different lines of the 2-dimensional data structure. For example, the lines may first be filled sequentially with each line being filled with a segment for the same time interval until all signals have been mapped, followed by the segment of all signals for the subsequent time interval being mapped to the consecutive lines, etc.

Different approaches may in different embodiments be used to determine the exact mapping/arrangement of the samples of the biomedical waveform time domain signal into the N-dimensional data structure.

For example, in some embodiments, the adapter 209 may evaluate a number of possible candidate arrangings/mappings and select the best arranging/mapping in accordance with a suitable measure. The adapter 209 may then control the mapping circuit 203 and the compressor 205 to use the arranging/mapping with the selected value. Thus, in some cases, a brute force approach may be used where a number of target mappings are evaluated and the one resulting in the best performance is selected.

In particular, in some embodiments, the adapter 209 control the mapping circuit 203 and the compressor 205 to generate compressed data for a plurality of different values of at least one parameter of the arranging/mapping. It may then proceed to determine a quality measure for the compressed data for each of the plurality of different values. For example, it may reconstruct the biomedical waveform time domain signal based on the compressed data for each of the different values and compare that to the original biomedical waveform time domain signal with the quality measure reflecting how well the reconstructed biomedical waveform time domain signal matches the original biomedical waveform time domain signal. The adapter 207 may then proceed to select the value that results in the best quality measure. The adapter 207 may then control the mapping circuit 203 to use the arranging/mapping with the selected value. Thus, in some cases, a brute force approach may be used where a number of target mappings are evaluated and the one resulting in the best performance is selected.

In some embodiments, the adapter 209 may be arranged to analyze the biomedical waveform time domain signal to determine a suitable property/characteristic. The mapping/arranging may then be selected as one that is suitable for the determined value. For example, a number of different parameter values for the mapping/arranging may be stored and linked to values of a given property/characteristic. The parameter value matching the determined property value may then be selected and applied.

In some embodiments, the adapter 209 may specifically be arranged to determine the property of biomedical waveform time domain signal by an analysis that includes applying a continuous wavelet transform to the biomedical waveform time domain signal. The continuous wavelet transform may specifically be a (typically fast) continuous wavelet transform.

The continuous wavelet transform may determine a set of continuous wavelet transform coefficients from which the property may be extracted.

The continuous wavelet transform coefficients in particular represent information on the periodicity of the biomedical waveform time domain signal, and in particular with this being determined for different frequencies. Thus, the approach may provide data representing a time-frequency dependent frequency/periodicity of the biomedical waveform time domain signal.

The use of a (fast) continuous wavelet transform may in many embodiments provide a particularly advantageous information for the described compression approach. Further, it may in many embodiments be used to improve the compression process itself.

In particular, in many embodiments, the continuous wavelet transform may provide results that are directly used to adapt a wavelet transform which is part of the N-dimensional transform coding. For example, it may be used to adapt a level or depth of the wavelet transform of the (discrete) wavelet transform coding.

In particular, in many embodiments, the results of the (fast) continuous wavelet transform may be used to adapt/select the mother and/or father wavelet of the wavelet N-dimensional transform coding.

In some embodiments, some of the processing of the initial (fast) continuous wavelet transform and of the wavelet transform of the wavelet N-dimensional transform coding may be the same, and accordingly the generated results from the (fast) continuous wavelet transform may be used directly in the wavelet transform of the wavelet N-dimensional transform coding.

In more detail, the Continuous Wavelet Transform (CWT) provides a time-frequency representation of signals, allowing for detailed analysis of non-stationary signals with time-varying frequencies content like the biomedical waveform time domain signal (e.g., variations of the heartbeat period etc.). It decomposes a signal into scaled and shifted versions of a mother wavelet, enabling multi-resolution analysis and making it suitable for various applications in signal processing just like the DWT does. In particular, the approach may exploit complementarity of the CWT with respect to the DWT which enables it to analyze the frequency content of the biomedical waveform time domain signal with more finely captured details than with what is typically possible with the discrete wavelet technique DWT. Moreover, the CWT allows for a fast and finer determination of the periodicities present in the original biomedical waveform signal since it has the same time resolution as the original data in each frequency band (see Figs. 5 and 6). In particular, it is useful for the approach that the CWT creates a time-frequency representation of the original biomedical waveform time domain signal with all the instantaneous frequencies of the original biomedical waveform time domain signal. This CWT representation then renders it feasible to follow the time variations of such frequencies as it happens in the biomedical waveform time domain signal. The approach may exploit this property to define and update e.g. the property of periodicity for different parts of the original biomedical waveform time domain signal with the result being used by arranging performed by the mapping circuit 203 (e.g. via the operation of the adapter 209).

In many embodiments, the N-dimensional transform encoding/decoding may include a discrete wavelet transform and the determination of the property of the biomedical waveform time domain signal may be determined by applying a continuous wavelet transform to the biomedical waveform time domain signal to generate a set of continuous wavelet transform coefficients and determining the property of the biomedical waveform time domain signal from the set of continuous wavelet transform coefficients. A property of the discrete wavelet transform of the N-dimensional transform coding being dependent on a property of/determined from the discrete wavelet transform.

The apparatus(es) may specifically be implemented in one or more suitably programmed processors. The different functional blocks may be implemented in separate processors and/or may e.g., be implemented in the same processor. An example of a suitable processor is provided in the following.

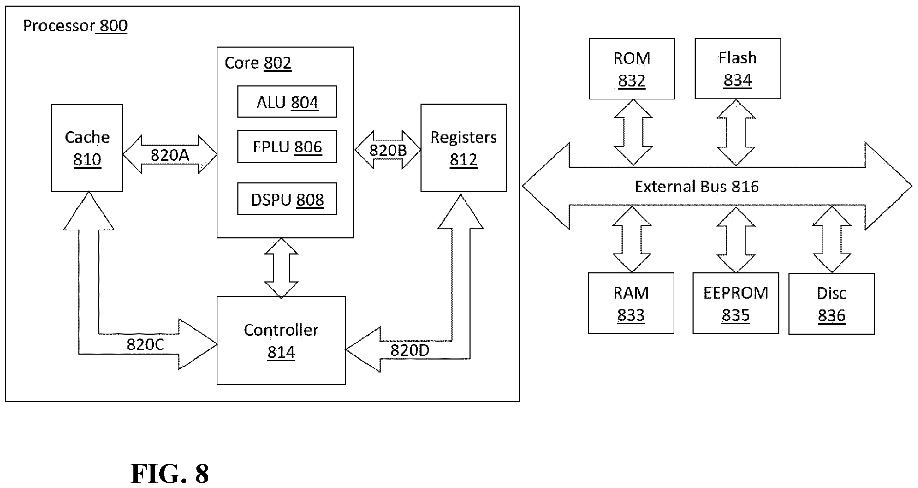

FIG. 8 is a block diagram illustrating an example processor 800 according to embodiments of the disclosure. Processor 800 may be used to implement one or more processors implementing an apparatus as previously described or elements thereof (including in particular one more artificial neural network). Processor 800 may be any suitable processor type including, but not limited to, a microprocessor, a microcontroller, a Digital Signal Processor (DSP), a Field ProGrammable Array (FPGA) where the FPGA has been programmed to form a processor, a Graphical Processing Unit (GPU), an Application Specific Integrated Circuit (ASIC) where the ASIC has been designed to form a processor, or a combination thereof.

The processor 800 may include one or more cores 802. The core 802 may include one or more Arithmetic Logic Units (ALU) 804. In some embodiments, the core 802 may include a Floating Point Logic Unit (FPLU) 806 and/or a Digital Signal Processing Unit (DSPU) 808 in addition to or instead of the ALU 804.

The processor 800 may include one or more registers 812 communicatively coupled to the core 802. The registers 812 may be implemented using dedicated logic gate circuits (e.g., flip-flops) and/or any memory technology. In some embodiments the registers 812 may be implemented using static memory. The registers may provide data, instructions and addresses to the core 802.

In some embodiments, processor 800 may include one or more levels of cache memory 810 communicatively coupled to the core 802. The cache memory 810 may provide computer-readable instructions to the core 802 for execution. The cache memory 810 may provide data for processing by the core 802. In some embodiments, the computer-readable instructions may have been provided to the cache memory 810 by a local memory, for example, local memory attached to the external bus 816. The cache memory 810 may be implemented with any suitable cache memory type, for example, Metal-Oxide Semiconductor (MOS) memory such as Static Random Access Memory (SRAM), Dynamic Random Access Memory (DRAM), and/or any other suitable memory technology.

The processor 800 may include a controller 814, which may control input to the processor 800 from other processors and/or components included in a system and/or outputs from the processor 800 to other processors and/or components included in the system. Controller 814 may control the data paths in the ALU 804, FPLU 806 and/or DSPU 808. Controller 814 may be implemented as one or more state machines, data paths and/or dedicated control logic. The gates of controller 814 may be implemented as standalone gates, FPGA, ASIC or any other suitable technology.

The registers 812 and the cache 810 may communicate with controller 814 and core 802 via internal connections 820A, 820B, 820C and 820D. Internal connections may be implemented as a bus, multiplexer, crossbar switch, and/or any other suitable connection technology.

Inputs and outputs for the processor 800 may be provided via a bus 816, which may include one or more conductive lines. The bus 816 may be communicatively coupled to one or more components of processor 800, for example the controller 814, cache 810, and/or register 812. The bus 816 may be coupled to one or more components of the system.

The bus 816 may be coupled to one or more external memories. The external memories may include Read Only Memory (ROM) 832. ROM 832 may be a masked ROM, Electronically Programmable Read Only Memory (EPROM) or any other suitable technology. The external memory may include Random Access Memory (RAM) 833. RAM 833 may be a static RAM, battery backed up static RAM, Dynamic RAM (DRAM) or any other suitable technology. The external memory may include Electrically Erasable Programmable Read Only Memory (EEPROM) 835. The external memory may include Flash memory 834. The external memory may include a magnetic storage device such as disc 836. In some embodiments, the external memories may be included in a system.

The invention can be implemented in any suitable form including hardware, software, firmware or any combination of these. The invention may optionally be implemented at least partly as computer software running on one or more data processors and/or digital signal processors. The elements and components of an embodiment of the invention may be physically, functionally and logically implemented in any suitable way. Indeed the functionality may be implemented in a single unit, in a plurality of units or as part of other functional units. As such, the invention may be implemented in a single unit or may be physically and functionally distributed between different units, circuits and processors.

Although the present invention has been described in connection with some embodiments, it is not intended to be limited to the specific form set forth herein. Rather, the scope of the present invention is limited only by the accompanying claims. Additionally, although a feature may appear to be described in connection with particular embodiments, one skilled in the art would recognize that various features of the described embodiments may be combined in accordance with the invention. In the claims, the term comprising does not exclude the presence of other elements or steps.

Furthermore, although individually listed, a plurality of means, elements, circuits or method steps may be implemented by e.g., a single circuit, unit or processor. Additionally, although individual features may be included in different claims, these may possibly be advantageously combined, and the inclusion in different claims does not imply that a combination of features is not feasible and/or advantageous. Also, the inclusion of a feature in one category of claims does not imply a limitation to this category but rather indicates that the feature is equally applicable to other claim categories as appropriate. Furthermore, the order of features in the claims does not imply any specific order in which the features must be worked and in particular the order of individual steps in a method claim does not imply that the steps must be performed in this order. Rather, the steps may be performed in any suitable order. In addition, singular references do not exclude a plurality. Thus references to "a", "an", "first", "second" etc. do not preclude a plurality. Reference signs in the claims are provided merely as a clarifying example shall not be construed as limiting the scope of the claims in any way.

## Claims

1. An apparatus for generating a data signal representing a biomedical waveform time domain signal, the apparatus comprising:
a receiver (201) arranged to receive the biomedical waveform time domain signal;
a circuit (203) arranged to arrange samples of the biomedical waveform time domain signal into an N-dimensional data structure, N being no less than two;
a compression circuit (205) arranged to generate compressed data representing the biomedical waveform time domain signal by applying a compression process to the N-dimensional data structure, the compression process including an N-dimensional transform coding;
a data signal generator (207) arranged to generate the data signal to include the compressed data; and
an adapter (209) arranged to adapt the arranging of samples of the biomedical waveform time domain signal into the N-dimensional data structure dependent on a property of the biomedical waveform time domain signal.

2. The apparatus of claim 1 wherein the property of the biomedical waveform time domain signal is a periodicity property of the biomedical waveform time domain signal.

3. The apparatus of any previous claim wherein the adapter (209) is arranged to adapt a property of the N-dimensional data structure dependent on the property of the biomedical waveform time domain signal.

4. The apparatus of any previous claim wherein the adapter (209) is arranged to adapt a mapping of the samples of the biomedical waveform time domain signal into the N-dimensional data structure dependent on the property of the biomedical waveform time domain signal.

5. The apparatus of any previous claim wherein the circuit (203) is arranged to map data samples of a segment of the biomedical waveform time domain signal into a slice of elements of a first dimension of the N-dimensional data structure.

6. The apparatus of claim 5 wherein the circuit (203) is arranged to adapt the mapping of the samples of the segment into the slice in dependence on a size of the N-dimensional data structure in the first dimension.

7. The apparatus of claim 6 wherein adapting the mapping includes at least one of:
- resampling the samples of the segment to generate a number of samples matching a number of elements in the slice;
- mapping only a subset of the samples of the segment into the slice; or
- mapping the samples of the segment into a subset of elements of the slice.

8. The apparatus of any of claims 5 to 7 wherein the adapter (209) is arranged to determine the segment in dependence on the property of the biomedical waveform time domain signal.

9. The apparatus of any previous claim wherein N=2 and the N-dimensional transform coding is an image transform coding.

10. The apparatus of any previous claim wherein the receiver is arranged to receive a further biomedical waveform time domain signal and the circuit (203) is arranged to map samples of the second biomedical waveform time domain signal into the N-dimensional data structure.

11. The apparatus of any previous claim wherein the adapter (209) is arranged to apply a continuous wavelet transform to the biomedical waveform time domain signal to generate a set of continuous wavelet transform coefficients, and to determine the property of the biomedical waveform time domain signal from the set of continuous wavelet transform coefficients.

12. The apparatus of 11 wherein the N-dimensional transform coding comprises a wavelet transform and the adapter (209) is arranged to adapt a property of the wavelet transform in dependence on the property.

13. The apparatus of any previous claim wherein the N-dimensional transform coding is a wavelet transform coding employing an N-dimensional wavelet.

14. The apparatus of any previous signal wherein the data signal generator (207) is arranged to include an indication of the adaptation in the data signal.

15. An apparatus for generating a reconstructed biomedical waveform time domain signal, the apparatus comprising:
a receiver (301) arranged to receive a data signal from a remote source, the data signal comprising compressed data representing a biomedical waveform time domain signal, the compressed data comprising a representation of an N-dimensional data structure;
a decompression circuit (303) arranged to determine the N-dimensional data structure from the compressed data by applying a decompression process to the compressed data, the compression process including an N-dimensional transform decoding;
a circuit (305) arranged to generate the reconstructed biomedical waveform time domain signal including arranging data of the N-dimensional data structure into samples of the reconstructed biomedical waveform time domain signal; and
an adapter (309) arranged to adapt the arranging data of the N-dimensional data structure into samples of the reconstructed biomedical waveform time domain signal depending on an indication comprised in the data signal, the indication being indicative of a property of an arranging by the remote source of samples of the biomedical waveform time domain signal into the N-dimensional data structure.

16. A method of generating a data signal representing a biomedical waveform time domain signal, the method comprising:
receiving the biomedical waveform time domain signal;
arranging samples of the biomedical waveform time domain signal into an N-dimensional data structure, N being no less than two;
generating compressed data representing the biomedical waveform time domain signal by applying a compression process to the N-dimensional data structure, the compression process including an N-dimensional transform coding;
generating the data signal to include the compressed data; and
adapting the arranging of samples of the biomedical waveform time domain signal into the N-dimensional data structure dependent on a property of the biomedical waveform time domain signal.

17. A method of generating a reconstructed biomedical waveform time domain signal, the method comprising:
receiving a data signal from a remote source, the data signal comprising compressed data representing a biomedical waveform time domain signal, the compressed data comprising a representation of an N-dimensional data structure;
determining the N-dimensional data structure from the compressed data by applying a decompression process to the compressed data, the compression process including an N-dimensional transform decoding;
generating the reconstructed biomedical waveform time domain signal including arranging data of the N-dimensional data structure into samples of the reconstructed biomedical waveform time domain signal; and
adapting the arranging data of the N-dimensional data structure into samples of the reconstructed biomedical waveform time domain signal depending on an indication comprised in the data signal, the indication being indicative of a property of an arranging by the remote source of samples of the biomedical waveform time domain signal into the N-dimensional data structure.
